(19) Europäisches Patentamt
European Patent Office
Office européen des brevets

(11) Veröffentlichungsnummer : **0 092 173**
**B1**

# (12) EUROPÄISCHE PATENTSCHRIFT

(45) Veröffentlichungstag der Patentschrift : **05.03.86**

(51) Int. Cl.[4] : **C 07 J 1/00**, A 61 K 31/565

(21) Anmeldenummer : **83103604.1**

(22) Anmeldetag : **14.04.83**

## (54) 11Beta-Chlor-Steroide, Verfahren zu ihrer Herstellung und diese enthaltende pharmazeutische Präparate.

(30) Priorität : **16.04.82 DE 3214689**

(43) Veröffentlichungstag der Anmeldung : **26.10.83 Patentblatt 83/43**

(45) Bekanntmachung des Hinweises auf die Patenterteilung : **05.03.86 Patentblatt 86/10**

(84) Benannte Vertragsstaaten : **AT BE CH DE FR GB IT LI LU NL SE**

(56) Entgegenhaltungen :
**FR-A- 1 566 154**
**NL-A- 7 209 299**
**US-A- 3 665 021**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Hofmeister, Helmut, Dr.**
**Weislingenstrasse 4**
**D-1000 Berlin 28 (DE)**
Erfinder : **Petzoldt, Karl, Dr.**
**Flachsweg 10**
**D-1000 Berlin 38 (DE)**
Erfinder : **Annen, Klaus, Dr.**
**Seegefelderstrasse 194**
**D-1000 Berlin 20 (DE)**
Erfinder : **Laurent, Henry, Dr.**
**Glambecker Weg 21**
**D-1000 Berlin 28 (DE)**
Erfinder : **Wiechert, Rudolf, Prof. Dr.**
**Petzowerstrasse 8 A**
**D-1000 Berlin 39 (DE)**
Erfinder : **Nishino, Yukishige, Dr.**
**Lanzendorfer Weg 14**
**D-1000 Berlin 22 (DE)**

EP 0 092 173 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft 11β-Chlor-Steroide, Verfahren zu ihrer Herstellung und diese enthaltende pharmazeutische Präparate.

Die neuen 11β-Chlor-Steroide werden durch die allgemeine Formel I gekennzeichnet

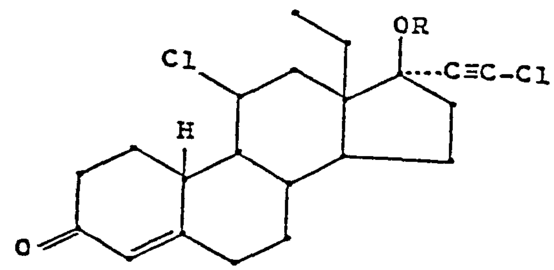

(I)

worin R ein Wasserstoffatom oder eine Acylgruppe einer organischen Carbonsäure mit bis zu 15 Kohlenstoffatomen bedeutet.

Es ist bereits bekannt, daß 11β-Chlor-Steroide wertvolle biologische Eigenschaften besitzen. In der niederländischen Offenlegungsschrift 7209299 werden zum Beispiel 11β-Chlor-17α-ethinyl-18-methyl-$\Delta^4$-östrene beschrieben, die eine starke gestagene Wirkung zeigen.

Es wurde nun gefunden, daß die noch nicht beschriebenen entsprechenden 17α-Chlorethinylverbindungen der allgemeinen Formel I etwa 3 mal stärker gestagen wirksam sind als die aus der NL-A-7209299 bekannten 17α-Ethinylverbindungen. Eine etwa gleiche Verstärkung der gestagenen Wirksamkeit erfahren die in FR-A-1566154 beschriebenen 11β-Des-Chlorverbindungen durch Einführung des 11β-Chlorsubstituenten.

Die gestagene Wirkung wurde im üblichen Clauberg-Test nach oraler Applikation an infantilen weiblichen Kaninchen ermittelt.

Die zur Erzielung eines positiven Effekts benötigte Mindestmenge wird durch den McPhail-Wert 1,5 ausgedrückt.

In der folgenden Tabelle werden die Ergebnisse des Tests zusammengestellt.

Tabelle

| | Verbindung | Dosis (mg) | McPhail |
|---|---|---|---|
| A | 11ß-Chlor-17α-chlorethinyl-17ß-hydroxy-18-methyl-4-östren-3-on | 0,1 | 3,3 |
| | | 0,03 | 3,0 |
| | | 0,01 | 1,9 |
| | | 0,003 | 1,2 |
| B | 11ß-Chlor-17α-ethinyl-17ß-hydroxy-18-methyl-4-östren-3-on | 0,03 | 2,5 |
| | | 0,01 | 1,4 |
| | | 0,003 | 1,1 |

Aus der Tabelle geht hervor, daß die Schwellendosis (McPhail 1,5) für die gestagene Wirkung bei der erfindungsgemäßen Verbindung A etwa bei 0,003 mg und bei der bekannten Verbindung B etwa bei 0,01 mg liegt.

Weiterhin besitzt A nur eine ganz geringfügige androgene Nebenwirkung, wie der hohe Kompetitionsfaktor $K_F = 15$ im Androgen-Rezeptor-Test zeigt. Demgegenüber ist das Steroid B, für das der Kompetitionsfaktor im gleichen Test $K_F = 6{,}3$ beträgt, noch schwach androgen wirksam.

Aufgrund der gestagenen Wirkung können die Verbindungen der allgemeinen Formel I zum Beispiel in Antikonzeptionspräparaten Verwendung finden, wobei sie als Gestagenkomponente in Kombination mit einer östrogen wirksamen Hormonkomponente, wie zum Beispiel Ethinylöstradiol, oder als alleinige Wirkkomponente eingesetzt werden. Die Verbindungen können aber auch in Präparaten zur Behandlung gynäkologischer Störungen verwendet werden.

Die neuen Verbindungen können mit den in der galenischen Pharmazie üblichen Zusätzen, Trägersubstanzen und Geschmackskorrigentien nach an sich bekannten Methoden zu den üblichen Arzneimittelformen verarbeitet werden. Für die orale Applikation kommen insbesondere Tabletten, Dragées, Kapseln, Pillen, Suspensionen oder Lösungen infrage. Für die parenterale Applikation kommen

insbesondere ölige Lösungen, wie zum Beispiel Sesamöl- oder Rizinusöllösungen infrage, die gegebenenfalls zusätzlich noch ein Verdünnungsmittel, wie zum Beispiel Benzylbenzoat oder Benzylalkohol, enthalten können. Die Konzentration des Wirkstoffes ist abhängig von der Applikationsform. So enthalten beispielsweise Tabletten zur oralen Applikation vorzugsweise 0,01-0,5 mg Wirkstoff und Lösungen zur parenteralen Applikation vorzugsweise 1-100 mg Wirkstoff pro 1 ml Lösung.

Die Dosierung der erfindungsgemäßen Arzneimittel kann sich mit der Form und dem Zweck der Verabfolgung ändern. Beispielsweise liegt die tägliche kontrazeptive Dosis bei oraler Applikation bei 0,01-0,5 mg des neuen Gestagens, gegebenenfalls in Kombination mit 0,01-0,05 mg Ethinylöstradiol.

Die Acylgruppen R gemäß Formel I leiten sich von Säuren ab, die in der Steroidchemie üblicherweise für Veresterungen angewendet werden. Bevorzugte Säuren sind organische Carbonsäuren mit bis zu 15 Kohlenstoffatomen, insbesondere niedere und mittlere aliphatische Carbonsäuren mit bis zu 7 Kohlenstoffatomen. Die Säuren können auch ungesättigt, verzweigt, mehrbasisch oder in üblicher Weise, zum Beispiel durch Hydroxy-, Acyloxy-, Alkoxy-, Oxo- oder Aminogruppen oder Halogenatome, substituiert sein. Geeignet sind auch cycloaliphatische, aromatische, gemischt aromatisch-aliphatische und heterocyclische Säuren, die ebenfalls in üblicher Weise substituiert sein können.

Beispielsweise seien folgende Carbonsäuren genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Tridecylsäure, Myristinsäure, Pentadecylsäure, Trimethylessigsäure, Diethylessigsäure, tert.-Butylessigsäure, β-Cyclopentylproionsäure, Cyclohexylessigsäure, Cyclohexancarbonsäure, Phenylessigsäure, Phenoxyessigsäure, Mono-, Di- und Trichloressigsäure, Aminoessigsäure, Diethylaminoessigsäure, Piperidinoessigsäure, Morpholinoessigsäure, Milchsäure, O-Tridecanoylglykolsäure, Bernsteinsäure, Adipinsäure, Benzoesäure, Nikotinsäure, Isonikotinsäure, Furan-2-carbonsäure.

Das in Anspruch 7 gekennzeichnete Verfahren wird nach an sich bekannten Methoden mit einer metallorganischen Chlorethinylverbindung durchgeführt, indem man das 17-Oxosteroid in einem geeigneten Lösungsmittel mit der metallorganischen Chlorethinylverbindung umsetzt. Die Chlorethinylverbindung wird in situ aus 1,2-Dichlorethylen und einer etherischen Alkylalkalimetall-Lösung, wie zum Beispiel Methyl- oder Butyllithiumlösung, gebildet. Geeignete Lösungsmittel sind Tetrahydrofuran und Diethylether.

Die 3-Ketogruppe wird zweckmäßigerweise vor der Chlorethinylierung geschützt. Die Oxoschutzgruppe Y der Formel II bildet mit der (den) Doppelbindung(en), welche im Ring A oder B vorhanden ist bzw. sind, eine solche Anordnung von Atomen, daß die Verbindung durch Hydrolyse zu einem 4,5-ungesättigten Keton umgewandelt wird. In einer bevorzugten Ausführungsform wird die Ketogruppe durch Ketalbildung geschützt. Die Ketalreste leiten sich von denüblicherweise zum Schutz freier Oxogruppen verwendeten Alkoholen und Thioalkoholen ab, beispielsweise genannt seien: Ethylenglykol, 2,2-Dimethyl-propandiol-(1,3) und Ethandithiol-(1,2). Die 3-Ketogruppe kann aber auch durch Enoläther-, Enolester- oder Enaminbildung partiell geschützt werden.

Die Abspaltung der 3-Ketoschutzgruppe, die vor oder auch nach der möglichen Veresterung erfolgen kann, wird nach den dem Fachmann bekannten Methoden durchgeführt. Zur Spaltung kommen Mineralsäuren, wie zum Beispiel Perchlorsäure, Schwefelsäure oder Salzsäure, oder organische Säuren, wie zum Beispiel Oxalsäure, in Betracht. Die Spaltung wird vorzugsweise in alkoholischer Lösung oder in anderen polaren Lösungsmitteln, wie zum Beispiel Aceton, bei Temperaturen zwischen etwa 20 und 100 °C durchgeführt.

Im Falle der Thioketalschutzgruppe erfolgt die Spaltung in alkoholischer Lösung, vorzugsweise in Methanol, mit Methyljodid unter Zugabe von Natrium- oder Kaliumacetat oder -carbonat bei Temperaturen zwischen Raumtemperatur und Siedetemperatur des Lösungsmittels.

Die sich gegebenenfalls anschließende Veresterung der 17-Hydroxygruppe erfolgt nach Methoden, die man üblicherweise in der Steroidchemie zur Veresterung tertiärer Hydroxygruppen anwendet. Als geeignete Veresterungsmethode sei Beispielsweise die Umsetzung der Steroide mit Säureanhydriden oder Säurechloriden in Gegenwart basischer Katalysatoren, wie Natriumhydrogenkarbonat, Kaliumhydrogenkarbonat, Kaliumkarbonat, Natriumhydroxid, Kaliumhydroxid, Pyridin, Lutidin, Collidin, Triethylamin oder 4-Dimethylaminopyridin, genannt. Nach einer bevorzugten Ausführungsform wird die Veresterung in Gegenwart von Pyridin und 4-Dimethylaminopyridin durchgeführt.

Die nach dem erfindungsgemäßen Verfahren eingesetzten Ausgangsverbindungen der allgemeinen Formel II können aus 11β-Chlor-18-methyl-4-östren-3,17-dion (niederländische Offenlegungsschrift 7209299) zum Beispiel durch Ketalisierung wie folgt hergestellt werden:

11β-Chlor-3,3'-(2',2'-dimethyl-trimethylendioxy)-18-methyl-5-östren-17-on

500 mg 11β-Chlor-18-methyl-4-östren-3,17-dion werden bei Raumtemperatur in 5 ml Methylenchlorid und 0,5 ml o-Ameisensäuretriethylester mit 1 g 2,2-Dimethyl-1,3-propandiol und 5 mg p-Toluolsulfonsäure versetzt. Nach 6 Stunden verdünnt man mit Methylenchlorid, wäscht neutral und trocknet. Das Rohprodukt wird an Kieselgel chromatographiert. Mit Aceton/Hexan werden 300 mg 11β-Chlor-3,3-(2',2'-dimethyl-trimethylendioxy-18-methyl-5-östren-17-on vom Schmelzpunkt 161,2 °C eluiert.

Beispiel 1

a) 11β-Chlor-17α-chlorethinyl-3,3-(2′,2′-dimethyl-trimethylendioxy)-18-methyl-5-östren-17β-ol

Zu 2,8 ml Dichlorethylen in 20 ml absolutem Ether werden unter Eis/Wasser-Kühlung und Einleiten von Argon 21 ml einer 5 %igen etherischen Methyllithiumlösung getropft. Nach 30 Minuten gibt man 1,2 g 11β-Chlor-3,3-(2′,2′-dimethyl-trimethylendioxy)-18-methyl-5-östren-17-on in 5 ml absolutem Tetrahydrofuran hinzu. Man läßt bei Raumtemperatur rühren, versetzt nach 20 Minuten mit gesättigter Ammoniumchloridlösung, verdünnt mit Ether, wäscht mit Wasser und trocknet. Nach Umkristallisieren aus Aceton/Hexan erhält man 1,1 g 11β-Chlor-17α-chlorethinyl-3,3-(2′,2′-dimethyl-trimethylendioxy)-18-methyl-5-östren-17β-ol vom Schmelzpunkt 184,2 °C.

b) 11β-Chlor-17α-chlorethinyl-17β-hydroxy-18-methyl-4-östren-3-on

1,0 g 11β-Chlor-17α-chlorethinyl-3,3-(2′,2′-dimethyltrimethylen-dioxy)-18-methyl-5-östren-17β-ol werden unter Argon bei Raumtemperatur in 20 ml Aceton mit 0,5 ml halbkonzentrierter Salzsäure versetzt. Nach 1 Stunde neutralisiert man mit $NaHCO_3$-Lösung, engt im Vakuum ein, löst den Rückstand in Essigester, wäscht mit Wasser und trocknet. Das Rohprodukt ergibt nach Umkristallisieren aus Aceton/Hexan 433 mg 11β-Chlor-17α-chlorethinyl-17β-hydroxy-18-methyl-4-östren-3-on vom Schmelzpunkt 239,2 °C.

Beispiel 2

17β-Acetoxy-11β-chlor-17α-chlorethinyl-18-methyl-4-östren-3-on

300 mg 11β-Chlor-17α-chlorethinyl-17β-hydroxy-18-methyl-4-östren-3-on in 4 ml Pyridin werden bei Raumtemperatur unter Zugabe von 50 mg 4-dimethylaminopyridin mit 2 ml Acetanhydrid gerührt. Das Gemisch wird nach 2 Stunden in essigsäurehaltiges Eis/Wasser gegeben. Das ausgefallene Produkt wird abgesaugt, in Methylenchlorid gelöst und mit Wasser gewaschen. Nach Chromatographieren des Rohproduktes an Kieselge mit Hexan/Aceton erhält man 190 mg17β-Acetoxy-11β-chlor-17α-chlorethinyl-18-methyl-4-östren-3-on als Schaum.

Beispiel 3

17β-Butyryloxy-11β-chlor-17α-chlorethinyl-18-methyl-4-östren-3-on

110 mg 11β-Chlor-17α-chlorethinyl-17β-hydroxy-18-methyl-4-östren-3-on in 1 ml Pyridin werden unter Zusatz von 100 mg 4-Dimethylaminopyridin mit 0,5 ml Buttersäureanhydrid bei Raumtemperatur gerührt. Nach 5 Stunden wird das Gemisch in Eis/Wasser gegeben und das Produkt mit Methylenchlorid extrahiert. Nach Chromatographieren des Rohproduktes an Kieselgel mit Hexan/Aceton erhält man 73 mg 17β-Butyryloxy-11β-chlor-17α-chlorethinyl-18-methyl-4-östren-3-on als öliges Produkt.

Beispiel 4

11β-Chlor-17α-chlorethinyl-17β-heptanoyloxy-18-methyl-4-östren-3-on

300 mg 11β-Chlor-17α-chlorethinyl-17β-hydroxy-18-methyl-4-östren-3-on in 3 ml Pyridin versetzt man bei Raumtemperatur mit 1,5 ml Önanthsäureanhydrid und 100 mg 4-Dimethylaminopyridin. Nach 24 Stunden gibt man die Lösung in Eis/Wasser und extrahiert mit Methylenchlorid. Nach Chromatographieren des Rohproduktes an Kieselgel werden 160 mg 11β-Chlor-17α-chlorethinyl-17β-heptanoyloxy-18-methyl-4-östren-3-on als Öl erhalten.

**Patentansprüche** (für die Vertragsstaaten : BE, CH, DE, FR, GB, IT, LI, LU, NL, SE)

1. 11β-Chlor-Steroide der allgemeinen Formel I

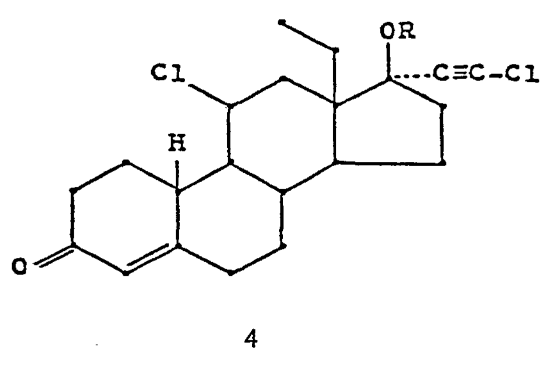

(I)

worin R ein Wasserstoffatom oder eine Acylgruppe einer organischen Carbonsäure mit bis zu 15 Kohlenstoffatomen bedeutet.

2. 11β-Chlor-17α-chlorethinyl-17β-hydroxy-18-methyl-4-östren-3-on.
3. 17β-Acetoxy-11β-chlor-17α-chlorethinyl-18-methyl-4-östren-3-on.
4. 17β-Butyryloxy-11β-chlor-17α-chlorethinyl-18-methyl-4-östren-3-on.
5. 11β-Chlor-17α-chlorethinyl-17β-heptanoyloxy-18-methyl-4-östren-3-on.
6. Pharmazeutische Präparate, gekennzeichnet durch einen Gehalt an Verbindungen gemäß Anspruch 1 bis 5.
7. Verfahren zur Herstellung von 11β-Chlor-Steroiden der allgemeinen Formel I

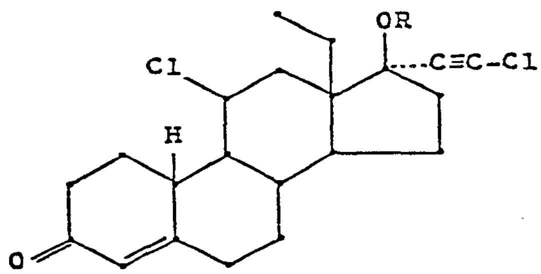

(I)

worin R ein Wasserstoffatom oder eine Acylgruppe einer organischen Carbonsäure mit bis zu 15 Kohlenstoffatomen bedeutet, dadurch gekennzeichnet, daß man ein 11β-Chlor-17-oxo-Steroid der allgemeinen Formel II

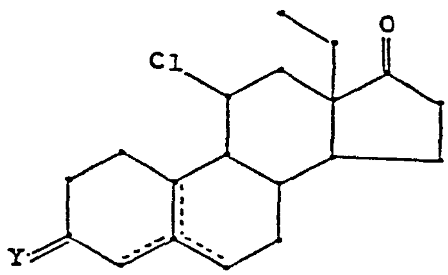

(II)

worin Y eine freie Oxogruppe oder eine hydrolysierbare Oxoschutzgruppe darstellt und

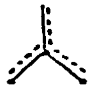

eine Doppelbindung in 4,5-, 5,6- oder 5,10-Stellung oder zwei von 3- und 5-Stellung ausgehende Doppelbindungen bedeutet,
nach an sich bekannten Methoden mit einer metallorganischen Chlorethinylverbindung zu einer Verbindung der allgemeinen Formel I, worin R ein Wasserstoffatom bedeutet, umsetzt, eine primär eingeführte 3-Oxoschutzgruppe hydrolysiert und, je nach der letztlich gewünschten Bedeutung von R, gegebenenfalls die 17-Hydroxygruppe vor oder nach der Abspaltung der Schutzgruppe verestert.

**Patentanspruch** (für den Vertragsstaat AT)

Verfahren zur Herstellung von 11β-Chlor-Steroiden der allgemeinen Formel I

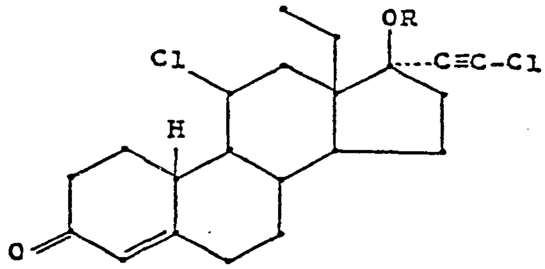

(I)

worin R ein Wasserstoffatom oder eine Acylgruppe einer organischen Carbonsäure mit bis zu 15 Kohlenstoffatomen bedeutet, dadurch gekennzeichnet, daß man ein 11β-Chlor-17-oxo-Steroid der allgemeinen Formel II

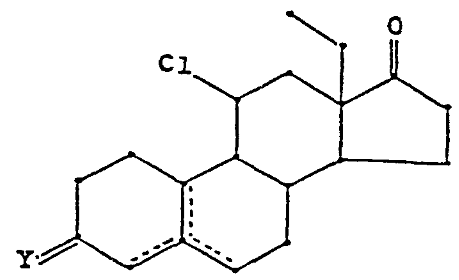

(II)

worin Y eine freie Oxogruppe oder eine hydrolysierbare Oxoschutzgruppe darstellt und

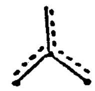

eine Doppelbindung in 4,5-, 5,6- oder 5,10-Stellung oder zwei von 3- und 5-Stellung ausgehende Doppelbindungen bedeutet,
nach an sich bekannten Methoden mit einer metallorganischen Chlorethinylverbindung zu einer Verbindung der allgemeinen Formel I, worin R ein Wasserstoffatom bedeutet, umsetzt, eine primär eingeführte 3-Oxoschutzgruppe hydrolysiert und, je nach der letztlich gewünschten Bedeutung von R, gegebenenfalls die 17-Hydroxygruppe vor oder nach der Abspaltung der Schutzgruppe verestert.

**Claims** (for the Contracting States : BE, CH, DE, FR, GB, IT, LI, LU, NL, SE)

1. 11β-Chlorosteroids of general formula I

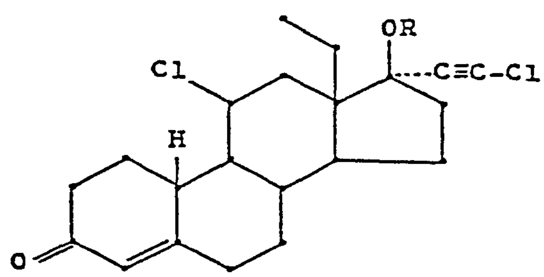

(I)

wherein R is hydrogen or an acyl group of an organic carboxylic acid of up to 15 carbon atoms.

2. 11β-Chloro-17α-chloroethynyl-17β-hydroxy-18-methyl-4-oestrene-3-one.

3. 17β-Acetoxy-11β-chloro-17α-chloroethynyl-18-methyl-4-oestrene-3-one.

4. 17β-Butyryloxy-11β-chloro-17α-chloroethynyl-18-methyl-4-oestrene-3-one.

5. 11β-Chloro-17α-chloroethynyl-17β-heptanoyloxy-18-methyl-4-oestrene-3-one.

6. Pharmaceutical compositions characterised in that they comprise a compound according to claims 1 to 5.

7. Process for the preparation of 11β-chlorosteroids of general formula I

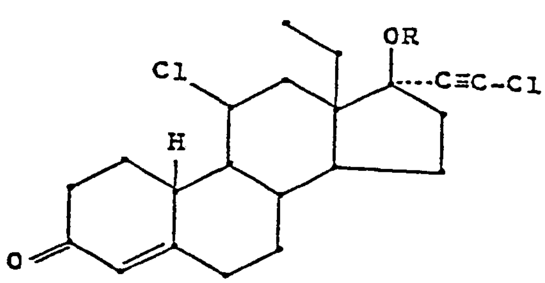

(I)

wherein R is hydrogen or an acyl group of an organic carboxylic acid of up to 15 carbon atoms characterised in that a 11β-chloro-17-oxosteroid of general formula II

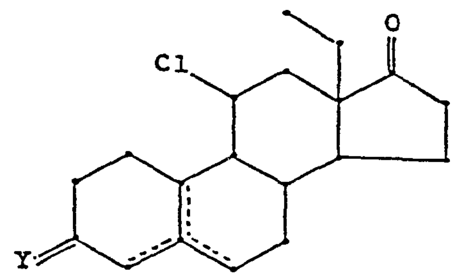

(II)

wherein, Y is a free oxo group or a hydrolysable protected oxo group and

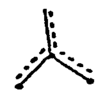

is a double bond in the 4,5-, 5,6- or 5,10-positions or two double bonds coming from the 3- and 5-positions, is converted, in a known manner, with an organometallic chloroethynyl compound to a compound of general formula I in which R is hydrogen, a primary introduced 3-oxo protecting group is hydrolysed and depending on the finally desired meaning of R, the 17-hydroxy group is optionally esterified, before or after removal of the protecting group.

**Claim** (for the Contracting State AT)

Process for the preparation of 11β-chlorosteroids of general formula I

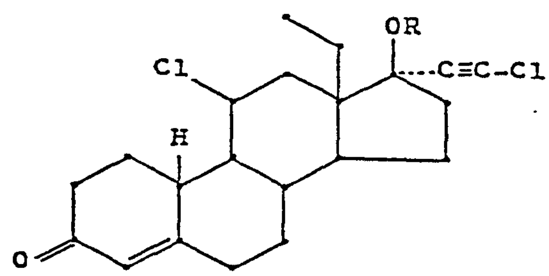

(I)

wherein R is hydrogen or an acyl group of an organic carboxylic acid of up to 15 carbon atoms characterised in that a 11β-chloro-17-oxosteroid of general formula II

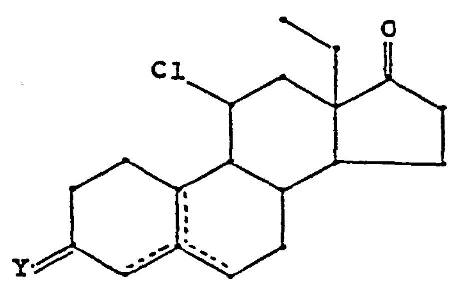

(II)

wherein, Y is a free oxo group of a hydrolysable protected oxo group and

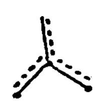

is a double bond in the 4,5-, 5,6- or 5,10-positions or two double bonds in the 3- and 5-positions, is converted, in a known manner, with an organometallic chloroethynyl compound to a compound of general formula I in which R is hydrogen, a primary introduced 3-oxo protecting group is hydrolysed and depending on the finally desired meaning of R, the 17-hydroxy group is optionally esterified, before or after removal of the protecting group.

**Revendications** (pour les Etats contractants : BE, CH, DE, FR, GB, IT, LI, LU, NL, SE)

1. 11β-chloro-stéroïdes répondant à la formule générale I

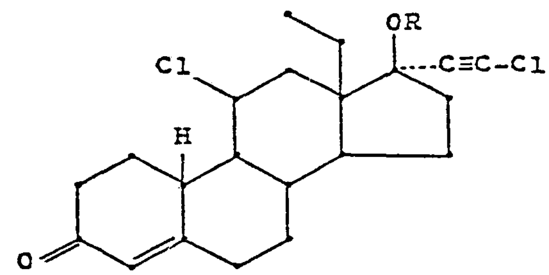

(I)

dans laquelle R représente un atome d'hydrogène ou un groupe acyle d'un acide carboxylique organique comprenant jusqu'à 15 atomes de carbone.

2. La 11β-chloro-17α-chloroéthynyl-17β-hydroxy-18-méthyl-4-œstrén-3-one.

3. La 17β-acétoxy-11β-chloro-17α-chloroéthynyl-18-méthyl-4-œstrén-3-one.

4. La 17β-butyryloxy-11β-chloro-17α-chloroéthynyl-18-méthyl-4-œstrén-3-one.

5. La 11β-chloro-17-chloroéthynyl-17β-heptanoyloxy-18-méthyl-4-œstrén-3-one.

6. Compositions pharmaceutiques, caractérisées par une teneur en des composés suivant l'une des revendications 1 à 5.

7. Procédé de préparation de 11β-chloro-stéroïdes répondant à la formule générale I

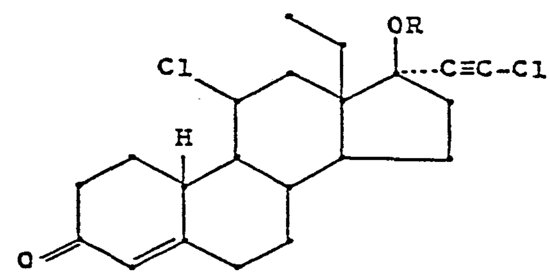

(I)

dans laquelle R représente un atome d'hydrogène ou un groupe acyle d'un acid carboxylique organique comprenant jusqu'à 15 atomes de carbone, caractérisé en ce qu'on fait réagir suivant des procédés connus en soi un 11β-chloro-17-oxo-stéroïde répondant à la formule générale II

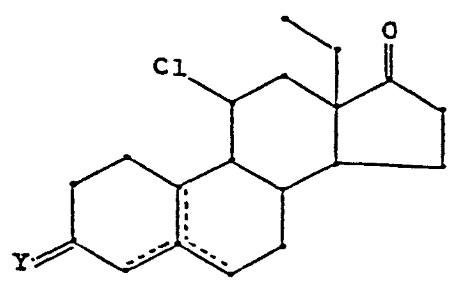

(II)

dans laquelle Y représente un groupe oxo libre ou un groupe de protection d'oxo hydrolysable, et

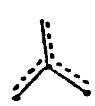

représente une double liaison en position 4,5, 5,6 ou 5,10 ou deux doubles liaisons partant de la position 3 et de la position 5,

avec un composé chloroéthynyle organométallique, ce qui donne un composé répondant à la formule générale I dans laquelle R représente un atome d'hydrogène, en ce qu'on hydrolyse un groupe de protection de 3-oxo introduit initialement et en ce que, selon la signification souhaitée à la fin pour R, on estérifie éventuellement le groupe 17-hydroxy avant ou après la séparation du groupe de protection.

**Revendication** (pour l'Etat contractant AT)

Procédé de préparation de 11β-chloro-stéroïdes répondant à la formule générale I

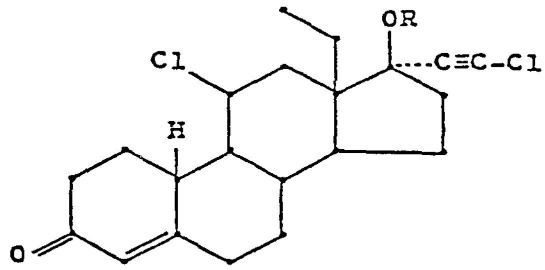

(I)

dans laquelle R représente un atome d'hydrogène ou un groupe acyle d'un acide carboxylique organique comprenant jusqu'à 15 atomes de carbone, caractérisé en ce qu'on fait réagir suivant des procédés connus en soi un 11β-chloro-17-oxo-stéroïde répondant à la formule générale II

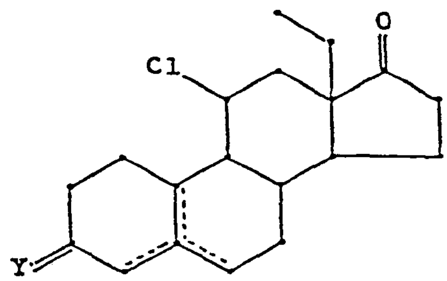

(II)

dans laquelle Y représente un groupe oxo libre ou un groupe de protection d'oxo hydrolysable,

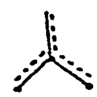

et représente une double liaison en position 4,5, 5,6 ou 5,10 ou deux doubles liaisons partant de la position 3 et de la position 5,
avec un composé chloroéthynyle organométallique, ce qui donne un composé répondant à la formule générale I dans laquelle R représente un atome d'hydrogène, en ce qu'on hydrolyse un groupe de protection de 3-oxo introduit initialement et en ce que, selon la signification souhaitée à la fin pour R, on estérifie éventuellement le groupe 17-hydroxy avant ou après la séparation du groupe de protection.